(19) Europäisches Patentamt
European Patent Office
Office européen des brevets

(11) **EP 0 851 848 B1**

(12) **FASCICULE DE BREVET EUROPEEN**

(45) Date de publication et mention de la délivrance du brevet:
**07.06.2000 Bulletin 2000/23**

(21) Numéro de dépôt: **96932566.1**

(22) Date de dépôt: **17.09.1996**

(51) Int Cl.[7]: **C07C 17/087**, C07C 19/08

(86) Numéro de dépôt international:
**PCT/EP96/04095**

(87) Numéro de publication internationale:
**WO 97/11042 (27.03.1997 Gazette 1997/14)**

(54) **PROCEDE DE PREPARATION D'HEPTAFLUOROPROPANE**

VERFAHREN ZUR HERSTELLUNG VON HEPTAFLUORPROPAN

METHOD FOR PREPARING HEPTAFLUOROPROPANE

(84) Etats contractants désignés:
**DE ES FR GB GR IT NL**

(30) Priorité: **20.09.1995 DE 19534917**

(43) Date de publication de la demande:
**08.07.1998 Bulletin 1998/28**

(73) Titulaire: **SOLVAY (Société Anonyme)
1050 Bruxelles (BE)**

(72) Inventeurs:
- **HOPP, Peter
D-65719 Hofheim (DE)**
- **KAUFMANN, Wolf-Dietmar
D-61476 Kronberg (DE)**

(74) Mandataire: **Jacques, Philippe et al
Solvay S.A.
Département Propriété Industrielle
310, rue de Ransbeek
1120 Bruxelles (BE)**

(56) Documents cités:
**EP-A- 0 634 383**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 851 848 B1

## Description

L'invention concerne un procédé de préparation continue de 1,1,1,2,3,3,3-heptafluoropropane (ci-après dénommé heptafluoropropane) par conversion d'hexafluoropropène avec du HF en présence d'un hydrofluorure liquide d'une base azotée organique répondant à la formule générale [B x nHF], où n représente un nombre entier ou une fraction $\leq 4$ et B représente une base azotée organique.

Dans le document EP-A-0 634 383, on décrit un procédé général d'addition de fluorure d'hydrogène sur des alcènes halogénés répondant à la formule générale $R^1CF{=}CR^2R^3$ avec un tel hydrofluorure [B x nHF], de préférence $[(CH_3)_3N \times 2{,}8\ HF]$ ou $[(C_2H_5)_3N \times 2{,}8\ HF]$, à titre de catalyseur. Les essais avec l'hexafluoropropène sont décrits à l'échelle de laboratoire, tant du point de vue discontinu que du point de vue continu. Dans le cas des deux variantes de réalisation, on n'obtient cependant pas une conversion complète. A cause de son prix élevé, l'hexafluoropropène résiduel contenu dans le produit final d'heptafluoropropane doit être récupéré par une distillation coûteuse.

L'objectif était de mettre au point un procédé continu pour la préparation d'heptafluoropropane avec une conversion complète de l'hexafluoropropène.

Dans le document EP-A-0 634 383 mentionné ci-dessus, on utilise dans les Exemples 4 et 5 une colonne à bulles (récipient de réaction avec du catalyseur d'hydrofluorure liquide qui est entièrement chargé de bulles de gaz des réactifs et du produit) pour la conversion en continu d'hexafluoropropène avec du HF. La conversion de l'hexafluoropropène s'élève respectivement à 98,4 et à 99 %. Bien que ces valeurs semblent relativement élevées, elles ne sont cependant pas suffisamment élevées pour rendre superflu le traitement par distillation mentionné ci-dessus.

On a maintenant trouvé que la conversion de l'hexafluoropropène peut être augmentée à au moins 99,99 % en faisant passer le mélange réactionnel par deux zones disposées en série, une pression plus élevée régnant dans la première zone par comparaison à celle dans la deuxième zone. À cause de la conversion presque quantitative une élimination par distillation du matériau de départ non-converti n'est pas nécessaire. En outre, le procédé conforme à la présente invention peut être réalisé à grande échelle sans problèmes, ce qui n'est pas possible aussi simplement dans le cas d'une colonne à bulles.

L'objet de la présente invention est un procédé de préparation continue d'heptafluoropropane par conversion d'hexafluoropropène avec du HF en présence d'au moins un hydrofluorure liquide d'une base azotée organique répondant à la Formule (I),

$$[B \times nHF] \qquad (I),$$

dans laquelle B représente une base azotée organique et n représente un nombre entier ou une fraction $\leq 4$, caractérisé en ce qu'on convertit dans un procédé cyclique du HF et de l'hexafluoropropène en présence d'un hydrofluorure liquide répondant à la Formule (I) dans une première zone sous une pression $p_1$ = 1,3 à 10 bar, en ce que le mélange réactionnel liquide est ensuite transféré dans une deuxième zone et qu'il s'y trouve sous une pression $p_2 < p_1$, où $p_1 - p_2 \geq 0{,}3$ bar et $p_2 \geq 1$ bar, en ce que l'heptafluoropropane résultant est évaporé et isolé du mélange réactionnel liquide, et en ce qu'ensuite le mélange réactionnel liquide restant est transféré, après addition d'hexafluoropropène et de HF frais, de nouveau dans la première zone.

La température de réaction dans la première zone s'élève de préférence à 40 à 100 °C, en particulier à 50 à 80 °C. Des hydrofluorures appropriés, liquides à ces températures, répondant à la Formule (I), sont par exemple le $[(CH_3)_3N \times 2{,}8\ HF]$, le $[(C_2H_5)_3N \times 2{,}8\ HF]$, le $[(C_4H_9)_3N \times 2{,}6\ HF]$, de même que

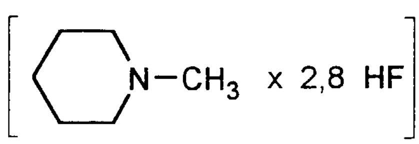,

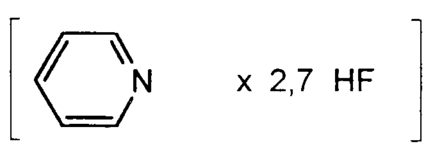

et

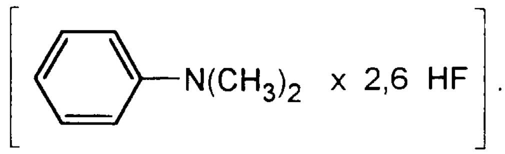

De préférence on utilise un des trois hydrofluorures mentionnés en premier lieu. Mais également tous les autres hydrofluorures répondant à la Formule (I), mentionnés dans le document EP-A-0 634 383, qui sont liquides aux températures de réaction choisies, sont appropriés. La préparation des hydrofluorures est également décrite dans le document EP-A-0 634 383, auquel on fait ici référence explicitement. Outre l'hexafluoropropène, les alcènes halogénés indiqués dans ce document peuvent être convertis d'une manière analogue au présent procédé, procédé au cours duquel ils réagissent avec un rendement environ aussi élevé que celui de l'hexafluoropropène.

Dans la première zone, on règle une pression $p_1$ = 1,3 à 10 bar, de préférence $p_1$ = 1,5 à 5 bar. Dans la deuxième zone, on règle une pression $p_2 < p_1$, où $p_2 \geq 1$ bar et la différence de pression $p_1 - p_2 \geq 0{,}3$ bar. De préférence la différence de pression $p_1 - p_2$ = 0,3 à 2 bar, ce qui peut toujours être réglé par un choix approprié de $p_1$ et de $p_2$ à l'intérieur des limites indiquées.

L'heptafluoropropane évaporé et isolé, ou soutiré par distillation, présente une pureté supérieure à 99 % avec un rendement supérieur à 99,99 %.

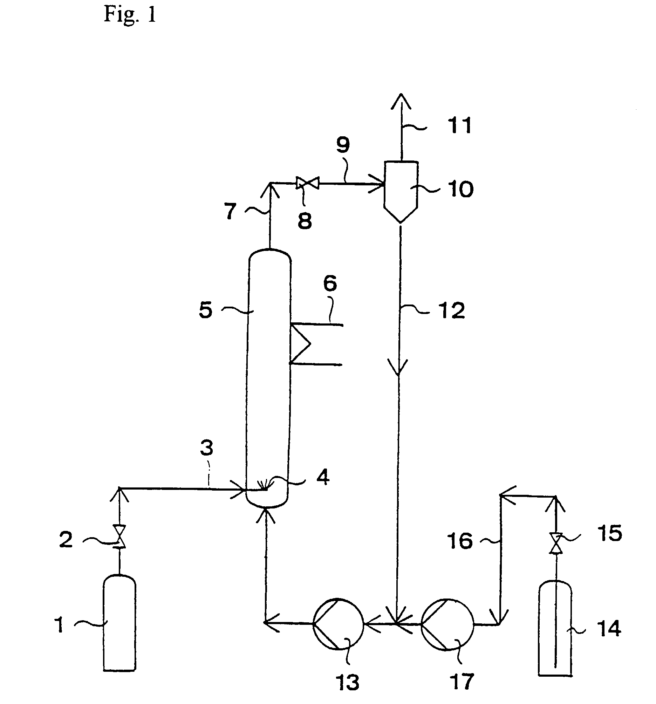

Le procédé conforme à la présente invention est réalisé par exemple dans l'appareillage suivant (Figure 1) :

De l'hexafluoropropène (HFP) est prélevé sous forme gazeuse du réservoir de stockage (1) et il est introduit par la vanne (2), par la conduite (3) et par le système de distribution de gaz (4) (par exemple des frittes de filtre, des filtres frittés, des fonds à tuyères) dans le réacteur (5) servant de première zone, qui est chauffé au moyen d'un système de chauffage (6). Le réacteur (5) est rempli avec le catalyseur d'hydrofluorure liquide. Par le système de distribution de gaz (4), le HFP est d'abord dispersé dans le catalyseur sous forme de petites bulles, qui se dissolvent rapidement d'une manière complète. La présence de bulles de gaz est limitée à la région inférieure du réacteur (environ 1/5 du volume total), de sorte qu'il ne s'agit pas d'une colonne à bulles (c'est-à-dire d'un réacteur chargé complètement de bulles de gaz).

Par la conduite (7), par la vanne (8) et par la conduite (9), le mélange réactionnel parvient au récipient (10) servant de deuxième zone et de cet endroit par la conduite (12) et par la pompe (13) de nouveau dans le réacteur (5). Par la conduite (11), on prélève le produit d'heptafluoropropane souhaité du récipient (10).

Au moyen de la vanne (8) (par exemple un obturateur ou une soupape), on règle dans le réacteur (5) la pression $p_1$ et dans le récipient (10) la pression $p_2 < p_1$.

Le HF est prélevé sous forme liquide du réservoir de stockage (14) et il est introduit par la vanne (15), par la conduite (16) et par la pompe (17) dans la conduite (12) et il parvient ensuite par la pompe (13) ensemble avec le catalyseur pompé par cette pompe dans le réacteur (5).

Exemple 1

On a travaillé dans l'appareillage décrit ci-dessus. Le réacteur (5) était constitué d'acier de type V4A (longueur = 3.000 mm, diamètre = 134,5 mm). À titre de catalyseur, on a utilisé du tris-hydrofluorure de tri-n-butylammonium (65 kg). La température dans le réacteur (5) s'élevait à 65 °C. La pression dans le réacteur (5) s'élevait à $p_1$ = 1,8 bar et dans le récipient (10) à $p_2$ = 1,0 bar.

Le débit massique s'élevait à : HF = 0,2 kg/h, HFP = 1,5 kg/h. À titre de système de distribution de gaz (4), on a utilisé des frittes de filtre (60 µm). La conversion du HFP s'élevait à 99,999 % et la pureté de l'heptafluoropropane obtenu était de 99,8 %.

Exemple 2

On a travaillé de la manière décrite à l'Exemple 1, avec la seule exception que le débit massique a été doublé : HF = 0,4 kg/h, HFP = 3 kg/h.

La conversion du HFP et la pureté de l'heptafluoropropane étaient tout aussi élevées qu'à l'Exemple 1.

Exemple 3

On a travaillé de la manière décrite à l'Exemple 1, avec la seule exception que le débit massique a été plus que triplé : HF = 0,65 kg/h, HFP = 5 kg/h.

La conversion du HFP et la pureté de l'heptafluoropropane étaient tout aussi élevées qu'à l'Exemple 1.

## Revendications

1. Procédé de préparation continue de 1,1,1,2,3,3,3-heptafluoropropane par conversion d'hexafluoropropène avec du HF en présence d'au moins un hydrofluorure liquide d'une base azotée organique répondant à la Formule (I),

$$[B \times nHF] \qquad (I),$$

dans laquelle B représente une base azotée organique et n représente un nombre entier ou une fraction ≤ 4, caractérisé en ce qu'on convertit dans un procédé cyclique du HF et de l'hexafluoropropène en présence d'un hydrofluorure liquide répondant à la Formule (I) dans une première zone sous une pression $p_1$ = 1,3 à 10 bar, en ce que le mélange réactionnel liquide est ensuite transféré dans une deuxième zone et qu'il s'y trouve sous une pression $p_2 < p_1$, où $p_1 - p_2 \geq 0{,}3$ bar et $p_2 \geq 1$ bar, en ce que l'heptafluoropropane résultant est évaporé et isolé du mélange réactionnel liquide, et en ce qu'ensuite le mélange réactionnel liquide restant est transféré, après addition d'hexafluoropropène et de HF, de nouveau dans la première zone.

2. Procédé selon la revendication 1, caractérisé en ce qu'on utilise à titre d'hydrofluorure répondant à la Formule (I) du $[(CH_3)_3N \times 2{,}8\ HF]$, du $[(C_2H_5)_3N \times 2{,}8\ HF]$ ou du $[(C_4H_9)_3N \times 2{,}6\ HF]$.

3. Procédé selon la revendication 1 ou 2, caractérisé en ce qu'on effectue la conversion dans la première zone à une température de 40 à 100 °C.

4. Procédé selon l'une des revendications 1 à 3, caractérisé en ce qu'on effectue la conversion dans la première zone à une pression $p_1$ = 1,5 à 5 bar.

5. Procédé selon l'une des revendications 1 à 4, caractérisé en ce qu'on choisit $p_1$ et $p_2$ de telle manière que $p_1 - p_2$ = 0,3 bar à 2 bar.

## Patentansprüche

1. Verfahren zur kontinuierlichen Herstellung von 1,1,1,2,3,3,3-Heptafluorpropan durch Umsetzen von Hexafluorpropen mit HF in Gegenwart wenigstens eines flüssigen Hydrofluorids einer organischen stickstoffhaltigen Base, das der Formel (I) entspricht,

$$[B \times nHF] \qquad (I),$$

in der B eine organische stickstoffhaltige Base darstellt und n eine ganze Zahl oder einen Bruch ≤ 4 darstellt, dadurch gekennzeichnet, dass man in einem cyclischen Verfahren HF und Hexafluorpropen in Gegenwart eines flüssigen Hydrofluorids, das der Formel (I) entspricht, in einer ersten Zone unter einem Druck $p_1$ = 1,3 bis 10 bar umsetzt, dadurch, dass das flüssige Reaktionsgemisch dann in eine zweite Zone überführt wird und dass es sich dort unter einem Druck $p_2 < p_1$ befindet, wobei $p_1 - p_2 \geq 0{,}3$ bar und $p_2 \geq 1$ bar, dadurch, dass das resultierende Heptafluorpropan aus dem flüssigen Reaktionsgemisch verdampft und isoliert wird, und dadurch, dass das verbleibende flüssige Reaktionsmedium dann nach Zugabe von Hexafluorpropen und HF von neuem in die erste Zone überführt wird.

2. Verfahren gemäß Anspruch 1, dadurch gekennzeichnet, dass man als Hydrofluorid, das der Formel (I) entspricht, $[(CH_3)_3N \times 2{,}8\ HF]$, $[(C_2H_5)_3N \times 2{,}8\ HF]$ oder $[(C_4H_9)_3N \times 2{,}6\ HF]$ verwendet.

3. Verfahren gemäß Anspruch 1 oder 2, dadurch gekennzeichnet, dass man die Umsetzung in der ersten Zone bei einer Temperatur von 40 bis 100 °C ausführt.

4. Verfahren gemäß einem der Ansprüche 1 bis 3, dadurch gekennzeichnet, dass man die Umsetzung in der ersten Zone bei einem Druck $p_1$ = 1,5 bis 5 bar ausführt.

**5.** Verfahren gemäß einem der Ansprüche 1 bis 4, dadurch gekennzeichnet, dass man $p_1$ und $p_2$ auf solche Weise wählt, dass $p_1 - p_2 = 0{,}3$ bar bis 2 bar.

## Claims

**1.** Process for the continuous preparation of 1,1,1,2,3,3,3-heptafluoropropane by conversion of hexafluoropropene with HF in the presence of at least one liquid hydrofluoride of an organonitrogen base corresponding to formula (I)

$$[B \times nHF] \quad \text{(I)},$$

in which B represents an organonitrogen base and n represents an integer or a fraction $\leq 4$, characterized in that HF and hexafluoropropene are converted, in a cyclic process, in the presence of a liquid hydrofluoride corresponding to formula (I), in a first zone under a pressure $p_1$ = 1.3 to 10 bar, in that the liquid reaction mixture is then transferred to a second zone and that it comes under a pressure $p_2 < p_1$ therein, in which $p_1 - p_2 \geq 0.3$ bar and $p_2 \geq 1$ bar, in that the resulting heptafluoropropane is evaporated and isolated from the liquid reaction mixture, and in that the remaining liquid reaction mixture is then transferred, after addition of hexafluoropropene and HF, back into the first zone.

**2.** Process according to Claim 1, characterized in that $[(CH_3)_3N \times 2.8\ HF]$, $[(C_2H_5)_3N \times 2.8\ HF]$ or $[(C_4H_9)_3N \times 2.6\ HF]$ is used as hydrofluoride corresponding to formula (I).

**3.** Process according to Claim 1 or 2, characterized in that the conversion in the first zone is carried out at a temperature of 40 to 100°C.

**4.** Process according to one of Claims 1 to 3, characterized in that the conversion in the first zone is carried out at a pressure $p_1$ = 1.5 to 5 bar.

**5.** Process according to one of Claims 1 to 4, characterized in that $p_1$ and $p_2$ are chosen such that $p_1 - p_2 = 0.3$ bar to 2 bar.

Fig. 1